# Europäisches Patentamt
# European Patent Office
# Office européen des brevets

(19)

(11) Numéro de publication: **0 092 451**
**B1**

(12) ## FASCICULE DE BREVET EUROPEEN

(45) Date de publication du fascicule du brevet:
14.08.85

(51) Int. Cl.⁴: **A 61 J 15/00**

(21) Numéro de dépôt: 83400562.1

(22) Date de dépôt: **17.03.83**

(54) Sonde d'alimentation entérale.

(30) Priorité: **08.04.82 FR 8206168**

(43) Date de publication de la demande:
**26.10.83 Bulletin 83/43**

(45) Mention de la délivrance du brevet:
**14.08.85 Bulletin 85/33**

(84) Etats contractants désignés:
**BE CH DE GB LI NL**

(56) Documents cités:
**FR - A - 1 129 401**
**FR - A - 2 190 479**
**FR - A - 2 436 557**
**US - A - 3 499 435**
**US - A - 3 818 895**
**US - A - 3 951 136**

**IBM TECHNICAL DISCLOSURE BULLETIN, vol. 11, no. 11, avril 1969, page 1565, New York, USA E.R. ELLENWOOD et al.: "Central-body temperature apparatus"**

(73) Titulaire: **Société VYGON, 5-11 rue Adeline, F-95440 Ecouen (FR)**

(72) Inventeur: **Stockman, Bernard, Domaine d'Amblaincourt, F-60230 Chambly (FR)**

(74) Mandataire: **Corre, Jacques Denis Paul et al, Cabinet Regimbeau 26, Avenue Kléber, F-75116 Paris (FR)**

## Description

La présente invention concerne une sonde d'alimentation entérale.

Les sondes d'alimentation entérale comportent, pour l'essentiel, un tube dont l'extrémité proximale est prévue pour être raccordée à un flacon d'alimentation et dont l'extrémité distale est destinée à être mise en place dans l'estomac d'un patient.

Dans de nombreux cas de pathologie infantile, il est nécessaire d'alimenter l'enfant 24 h sur 24 ou du moins de nombreuses fois dans la journée et dans la nuit.

Il est très difficile de surveiller un enfant alimenté par sonde de façon qu'il ne puisse retirer la sonde en place dans son estomac. Or, il est absolument essentiel que la sonde ne puisse quitter sa place, faute de quoi l'alimentation ne peut plus se poursuivre et il peut alors en résulter des troubles graves engendrés par l'interruption de la thérapie.

Les sondes d'alimentation entérale qui existent actuellement exigent une surveillance constante pour s'assurer que la sonde n'a pas quitté l'estomac du malade, surtout dans le cas où il s'agit d'un enfant.

La présente invention concerne une sonde d'alimentation entérale qui permet d'éviter les inconvénients ci-dessus et qui peut être utilisée sans qu'il soit nécessaire de veiller de façon permanente le malade et surtout l'enfant.

La présente invention concerne plus particulièrement une sonde d'alimentation entérale qui comporte un détecteur de température placé à l'intérieur du tube d'alimentation, et à distance de l'extrémité distale, de telle sorte que le détecteur de température se trouve normalement dans le corps humain et à la température humaine, ainsi qu'un avertisseur relié au détecteur de température et destiné à délivrer un signal dans le cas où la sonde quitte sa place en entraînant ainsi une chute de température au niveau du détecteur de température.

Selon une autre caractéristique de l'invention, le tube d'alimentation comprend un conduit principal d'alimentation et un conduit secondaire qui est disposé à l'intérieur du conduit principal et dans lequel sont placés le détecteur de température et des fils électriques de liaison à l'avertisseur.

Selon encore une autre caractéristique de l'invention, l'extrémité distale du conduit secondaire est fermée et le détecteur de température se trouve à une distance d'environ 20 cm de celle-ci.

Selon encore une autre caractéristique de l'invention, l'extrémité proximale du tube est munie d'un raccord à dérivation latérale comprenant une branche principale en liaison directe avec le conduit principal et une branche secondaire latérale en liaison avec le conduit secondaire.

Le détecteur de température est avantageusement constitué par une thermistance et l'avertisseur par un dispositif électronique produisant un signal sonore et un signal lumineux.

D'autres caractéristiques et avantages de l'invention seront mieux compris à la lecture de la description qui suit et qui se réfère au dessin annexé, donné uniquement à titre illustratif de l'invention, sur lequel:

la fig. 1 est une vue en élévation d'une sonde d'alimentation entérale selon l'invention, et

la fig. 2 est une vue partielle à échelle agrandie de la portion du tube de la sonde de la fig, 1 qui reçoit le détecteur de température.

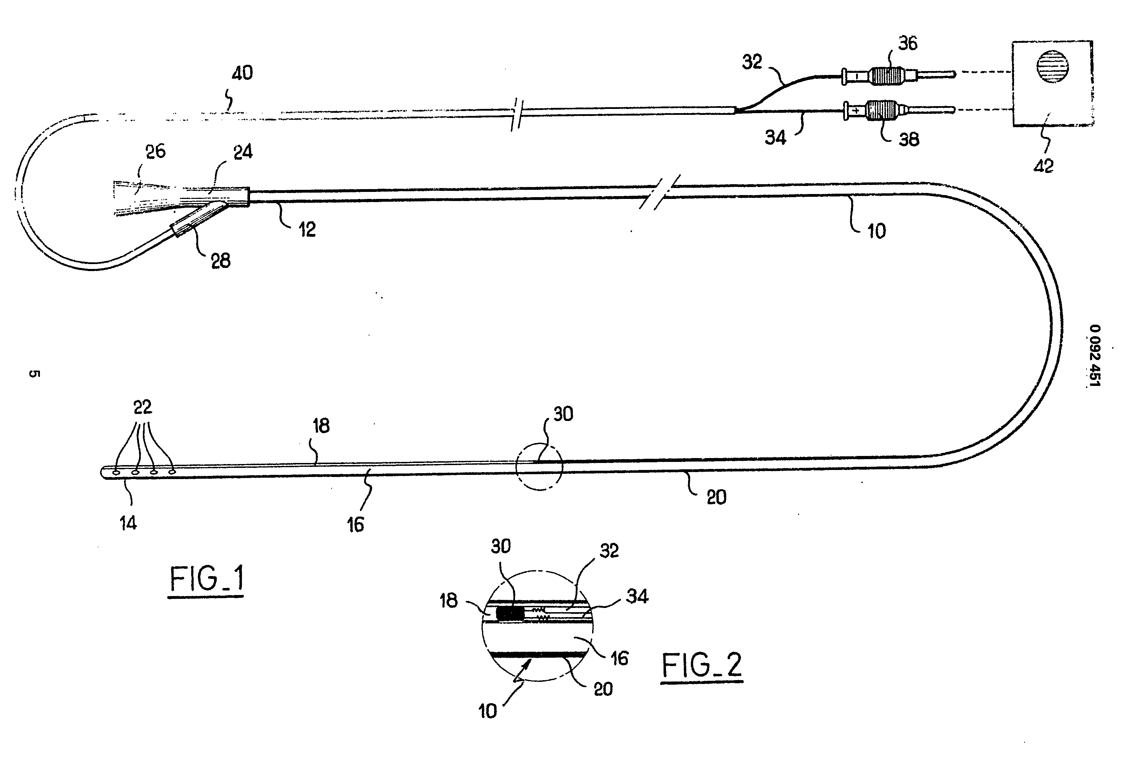

La sonde représentée sur la fig. 1 comporte un tube d'alimentation 10 dont l'extrémité proximale 12 est destinée à être raccordée à un flacon d'alimentation (non représenté) et dont l'extrémité distale 14 est destinée à être mise en place dans l'estomac du malade. Le tube d'alimentation 10 comprend un conduit principal 16 servant à l'alimentation du malade et un conduit secondaire 18 disposé à l'intérieur du conduit principal 16.

Le tube 10 est de préférence constitué d'une matière thermoplastique ou d'un élastomère. Le tube 10 comporte par ailleurs une ligne 20 opaque aux rayons X qui est incluse dans l'épaisseur de la paroi du tube, afin de permettre la visualisation par radiographie aux rayons X de la position correcte de la sonde dans l'estomac.

Le tube 10 présente, à son extrémité distale 14, des orifices 22 par lesquels s'écoulera le liquide nutritif provenant du flacon d'alimentation branché sur l'extrémité proximale 12. Les orifices 22 sont percés dans le conduit principal 16, dont l'extrémité distale peut être également ouverte. En revanche, l'extrémité distale du conduit secondaire 18 doit être impérativement fermée.

L'extrémité proximale 12 du tube 10 est munie d'un raccord 24 à dérivation latérale comprenant une branche principale 26 en liaison directe avec le conduit principal 16 et une branche secondaire 28 en liaison avec le conduit secondaire 18. La branche principale 26 est destinée à être raccordée au flacon d'alimentation.

A l'intérieur du conduit secondaire 18, à une distance d'environ 20 cm de l'extrémité distale 14, est placé un détecteur de température 30 constitué par une thermistance. Cette thermistance est reliée, par l'intermédiaire de deux fils de connexion électrique 32 et 34 à deux fiches électriques, respectivement 36 et 38. Les fils électriques 32 et 34 passent à travers la branche secondaire 28 du raccord 24 et à travers un tube 40 raccordé à cette branche secondaire.

Les fiches 36 et 38 sont reliées à un avertisseur 42 constitué par un dispositif électronique produisant un signal sonore et/ou un signal lumineux. L'avertisseur 42 est réglé de manière à produire un signal lorsque la température du détecteur n'est plus à la température du corps humain et se trouve donc à la température ambiante.

Lorsque la sonde est placée dans l'estomac du malade, la thermistance présente une certaine valeur de résistance du fait que celle-ci se trouve à l'intérieur du corps du malade et donc à la température humaine de 37°C. Si le malade retire la sonde en place dans son propre estomac, la thermistance ne se trouve plus à la température humaine de 37°C et va s'approcher progressivement de la température ambiante voisine, non plus de

37°, mais de 20°, en modifiant par là même la valeur ohmique de la thermistance.

L'avertisseur 42 qui est branché sur la sonde enregistrera alors la variation de cette valeur ohmique et enclenchera une alarme sonore et/ou visuelle, placée dans le bureau de surveillance du service hospitalier ou dans la chambre des parents si le malade à surveiller est un enfant soigné à domicile.

## Revendications

1. Sonde d'alimentation entérale comportant un tube (10) dont l'extrémité proximale (12) est prévue pour être raccordée à un flacon d'alimentation et dont l'extrémité distale (14) est destinée à être mise en place dans l'estomac d'un malade, caractérisée par le fait qu'elle comporte un détecteur de température (30) placé à l'intérieur du tube (10) et à distance de l'extrémité distale (14) de telle sorte que le détecteur de température (30) se trouve normalement dans le corps humain et à la température humaine, ainsi qu'un avertisseur (42) relié au détecteur de température (30) et destiné à délivrer un signal dans le cas où la sonde quitte sa place en entraînant une chute de température au niveau du détecteur de température (30).

2. Sonde d'alimentation entérale selon la revendication 1, caractérisée par le fait que le tube d'alimentation (10) comprend un conduit principal d'alimentation (16) et un conduit secondaire (18) qui est disposé à l'intérieur du conduit principal (16) et dans lequel sont placés le détecteur de température (30) et des fils électriques (32, 34) de liaison à l'avertisseur (42).

3. Sonde d'alimentation selon la revendication 2, caractérisée par le fait que l'extrémité distale du conduit secondaire (18) est fermée et que le détecteur de température (30) se trouve à une distance d'environ 20 cm de celle-ci.

4. Sonde d'alimentation entérale selon l'une des revendications 2 ou 3, caractérisée par le fait que l'extrémité proximale (12) du tube (10) est munie d'un raccord (24) à dérivation latérale comprenant une branche principale (26) en liaison directe avec le conduit principal (16) et une branche latérale 28 en liaison avec le conduit secondaire (18).

5. Sonde d'alimentation entérale selon l'une des revendications 1 à 4, caractérisée par le fait que le détecteur de température (30) est une thermistance.

6. Sonde d'alimentation entérale selon l'une des revendications 1 à 5, caractérisée par le fait que l'avertisseur (42) est un dispositif électronique produisant un signal sonore et/ou un signal lumineux.

## Claims

1. Enteral supply probe comprising a tube (10) whereof the proximal end (12) is provided to be connected to a supply bottle and whereof the distal end (14) is intended to be fitted in a patient's stomach, characterised by the fact that it comprises a temperature detector (30) located inside the tube (10) and at a distance from the distal end (14), so that the temperature detector (30) is normally located in the human body and at the human temperature, as well as an alarm (42) connected to the temperature detector (30) and intended to emit a signal if the probe is displaced thus causing a temperature drop at the temperature detector (30).

2. Enteral supply probe according to Claim 1, characterised by the fact that the supply tube (10) comprises a main supply pipe (16) and a secondary pipe (18) which is located inside the main pipe (16) and in which the temperature detector (30) and electrical wires (32, 34) for connection to the alarm (42) are located.

3. Supply probe according to Claim 2, characterised by the fact that the distal end of the secondary pipe (18) is closed and that the temperature detector (30) is located at a distance of approximately 20 cm from the latter.

4. Enteral supply probe according to one of Claims 2 or 3, characterised by the fact that the proximal end (12) of the tube (10) is provided with a union (24) having a lateral branch comprising a main branch (26) in direct connection with the main pipe (16) and a side branch (28) connected to the secondary pipe (18).

5. Enteral supply probe according to one of Claims 1 to 4, characterised by the fact that the temperature detector (30) is a thermistance.

6. Enteral supply probe according to one of Claims 1 to 5, characterised by the fact that the alarm (42) is an electronic device producing a sound and/or a light signal.

## Patentansprüche

1. Sonde für enterale Ernährung mit einem Tubus (10), dessen proximales Ende (12) dazu vorgesehen ist, mit einer Ernährungsflasche verbunden zu werden, und dessen distales Ende (14) dazu bestimmt ist, im Magen eines Kranken angeordnet zu werden, dadurch gekennzeichnet, dass sie einen Temperaturdetektor (30) enthält, der im Inneren des Tubus (10) und in einem Abstand vom distalen Ende (14) in der Weise angeordnet ist, dass sich der Temperaturdetektor (30) normalerweise im menschlichen Körper und auf dessen Körpertemperatur befindet, sowie einer, mit dem Temperaturdetektor (30) verbundenen, Meldeeinrichtung (42), die dazu bestimmt ist, ein Signal zu liefern, falls die Sonde ihren Platz verlässt, was einen Temperaturabfall auf der Höhe des Temperaturdetektors (30) nach sich zieht.

2. Sonde für enterale Ernährung nach Anspruch 1, dadurch gekennzeichnet, dass der Ernährungstubus (10) einen Haupternährungskanal (16) umfasst, und einen Sekundärkanal (18), der im Inneren des Hauptkanals (16) angeordnet ist und in dem der Temperaturdetektor (30) und die elektrischen Anschlussdrähte (32, 34) der Meldeeinrichtung (42) angeordnet sind.

3. Sonde für enterale Ernährung nach Anspruch 2, dadurch gekennzeichnet, dass der Sekundärkanal (18) an seinem distalen Ende verschlossen ist, und dass der Temperaturdetektor (30) sich in einem Abstand von etwa 20 cm von diesem befindet.

4. Sonde für enterale Ernährung nach einem der Ansprüche 2 oder 3, dadurch gekennzeichnet, dass das proximale Ende (12) des Tubus (10) mit einem Verbindungsstück (24) mit seitlicher Abzweigung versehen ist, das einen mit dem Hauptkanal (16) direkt verbundenen Hauptzweig (26) und einen mit dem Sekundärkanal (18) verbundenen Seitenzweig (28) umfasst.

5. Sonde für enterale Ernährung nach einem der Ansprüche 1 bis 4, dadurch gekennzeichnet, dass der Temperaturdetektor (30) ein Thermistor ist.

6. Sonde für enterale Ernährung nach einem der Ansprüche 1 bis 5, dadurch gekennzeichnet, dass die Meldeeinrichtung (42) eine elektronische Vorrichtung ist, die ein Tonsignal und/oder ein Leuchtsignal produziert.

FIG_1

FIG_2

0 092 451